# EUROPEAN PATENT SPECIFICATION

(11) **EP 2 826 500 B1**
(45) Date of publication and mention of the grant of the patent: **24.08.2016**
(21) Application number: 13761048.1
(22) Date of filing: 13.03.2013
(51) Int. Cl.: A61M 1/02, A61J 1/10, A61J 1/12

(54) **CONTAINER FOR TESTING BLOOD AND BLOOD SAMPLING INSTRUMENT**
BEHÄLTER ZUR UNTERSUCHUNG VON BLUT UND BLUTENTNAHMEVORRICHTUNG
RÉCIPIENT POUR ANALYSE DE SANG ET INSTRUMENT DE PRÉLÈVEMENT SANGUIN

(30) Priority: 14.03.2012 JP 2012057501
(43) Date of publication of application: 21.01.2015
(73) Proprietor: Terumo Kabushiki Kaisha, Tokyo 151-0072 (JP)
(72) Inventor: HAYAKAWA Aya, Fujinomiya-shi Shizuoka 418-0004 (JP); AKIYAMA Masahiro, Fujinomiya-shi Shizuoka 418-0004 (JP)
(74) Representative: TBK
(86) International application number: PCT/JP2013/057099
(87) International publication number: WO 2013/137359

(56) References cited:
- EP-A1- 2 394 678
- WO-A1-2011/093260
- WO-A2-03/041634
- JP-A- H11 197 236
- JP-A- 2009 100 868
- JP-B2- 4 839 198
- JP-B2- 4 879 704

## Description

### Technical Field

The present invention relates to a blood sample container for collecting (storing) an initial flow of blood (hereinafter referred to as initial flow blood) to be used for testing according to the preamble of claim 1, the features of which are known from e.g. document JP 4 839198 B2, and to a blood collecting instrument provided with the blood sample container.

### Background Art

A blood collecting instrument is normally provided with a vein puncture needle, a donor bag for storing blood collected by the vein puncture needle, a plastic pipe connecting the vein puncture needle and the donor bag together, a pipe segment branching from the middle of the plastic pipe, and a container (blood sample container) communicating with the plastic pipe via the pipe segment, as described in Patent Literature 1, for example.

In the usage of the blood collecting instrument of Patent Literature 1, initial flow blood of a donor (a person who donates his/her blood) is collected (drawn) from an entry port at a lower position of the blood sample container into an internal chamber of the blood sample container before blood of the donor is collected (stored) into the donor bag, in other words, before a blood sample of the donor is obtained. Further, the initial flow blood having been collected (stored) is collected (introduced) into a blood sampling vial communicating with an exit port at an upper position of the blood sample container, and is used for various tests.

The blood collecting instrument of Patent Literature 1 thus offers an advantage that initial flow blood to be used for various tests can be easily collected (stored). Another advantage is that blood to be collected (stored) into the donor bag can be prevented from being contaminated with bacteria which was present on or under the skin of a donor and entered blood when collected, because the blood contaminated with the bacteria can be eliminated as initial flow blood into an initial flow blood bag.

In the usage of the blood collecting instrument of Patent Literature 1, however, a worker (a person who collects blood) starts collection of initial flow blood into the blood sample container, visually confirms by the liquid level of the collected initial flow blood that a predetermined amount of initial flow blood has been collected into the blood sample container, closes a clamp set in the pipe segment, and stops (terminates) the drawing of the initial flow blood into the blood sample container. Since the drawing of the initial flow blood is manually stopped in the usage of the blood collecting instrument of Patent Literature 1, the timings of closing the clamp differ among workers, resulting in variations in the amount of initial flow blood collected (stored) in the blood sample container. Accordingly, initial flow blood in an amount excessively large or less than a required minimum is collected in some cases. When the amount of collected blood is too large, the medical conditions of the donor may be compromised or blood loss may occur. On the other hand, when the minimum amount cannot be obtained, various tests cannot be conducted with the initial flow blood and the safety of the blood collected in the donor bag cannot be assured. In particular, failure of collecting the necessary minimum amount of initial flow blood leads to inability to manufacture blood products using the blood collected with the blood collecting instrument, and shortage of blood products is consequently caused.

In order to solve the problems, Patent Literature 2 discloses a blood sample container with the following components. The blood sample container disclosed in Patent Literature 2 includes: a container body that stores initial flow blood to be used for testing; a blood inflow port communicating with the container body, that allows the initial flow blood to flow in; a blood collecting port having a communication port communicating with the container body, that collects the initial flow blood in the container body through the communication port; and an exhaust port having a hydrophobic filter liquid-impermeable and bacteria-impermeable, that allows air in the container body to be discharged, the exhaust port being provided at a position above the blood inflow port in a usage state where the initial flow blood is being introduced into the container body.

In the blood sample container with the configurations disclosed in Patent Literature 2, the liquid level of initial flow blood in the container body rises as the initial flow blood flows into the container body. When the amount of the initial flow blood in the container body reaches a minimum amount required, the initial flow blood gets in contact with the hydrophobic and bacteria-impermeable filter in almost all the surfaces of the filter and automatically stops flowing into the container body. Consequently, a predetermined amount of the initial flow blood including the required minimum amount can be stored in the container body.

### Citation List

### Patent Literatures

Patent Literature 1: Japanese Translation of PCT International Application Publication No. JP-T-2003-505185
Patent Literature 2: JP Patent Publication No. 4839198

### Summary of Invention

### Technical Problem

The invention disclosed in Patent Literature 2, however, suffers a disadvantage that workers have difficulty in realizing that blood collection has been automatically completed after the contact of initial flow blood with the hydrophobic and bacteria-impermeable filter, since the width of the blood sample container according to Patent Literature 2 is set to be relatively large and this reduces the rate at which the liquid level of the blood collected in the container rises. Likewise, the width of the blood sample container according to Patent Literature 1 is set to be relatively large and this reduces the rate at which the liquid level of blood collected in the container changes (rises), and therefore the invention of Patent Literature 1 suffers a disadvantage that workers cannot easily realize the timing of closing the clamp to complete the collection of initial flow blood.

Further, in recent blood collection, there has been demands for not only securing initial blood flow in a required minimum amount but also fine-adjusting the amount of initial blood flow to be collected at the level of several ml according to donors. The blood sample containers disclosed in Patent Literatures 1 and 2, however, cannot respond to the demands because of their specifications, with which a required minimum amount cannot be secured or the amount of blood to be collected is fixed and cannot be fine-adjusted if a required minimum amount can be secured, as described above.

The present invention has been made in order to solve the above problems, and the object of the present invention is to provide a blood sample container that enables workers to easily recognize completion of initial flow blood collection, secure a required minimum amount of initial flow blood, and fine-adjust the amount of initial flow blood to be collected at the level of several ml according to donors, and a blood collecting instrument provided with the blood sample container.

The object of the invention is achieved by a blood sample container according to claim 1. Advantageous embodiments are carried out according to the dependent claims.

### Solution to Problem

A blood sample container according to the present invention includes: a container body section having an internal space for storing initial flow blood to be used for testing; a blood inflow port communicating with the internal space, that allows the initial flow blood to flow in; a blood outflow port communicating with the internal space, that allows the stored initial flow blood to flow out; and an exhaust section communicating with the internal space and having a hydrophilic and bacteria-impermeable filter, that allows the air in the internal space to be discharged, in which the exhaust section is positioned at the upper end of the container body section when the blood sample container is used in a manner such that the blood outflow port faces in a vertical direction, and has a small-diameter tubular passage that allows the air in the internal space to be discharged by a predetermined amount of the initial flow blood that has flown into the passage. Further, the tubular passage of the blood sample container is desirably at least one of a first small-diameter tubular passage and a second small-diameter tubular passage, the first tubular passage being formed of a tube connected to the container body section and the second tubular passage being formed of an upward protruding portion of the internal space.

With the above configurations, since the exhaust section is arranged at the upper end of the container body section and includes the tubular passage for entry of initial flow blood in a predetermined amount, the air in the internal space is discharged by initial flow blood that has flown into the exhaust section at the end of the initial flow blood collection, and the inflow of the initial flow blood into the exhaust section causes a change in the liquid level in the exhaust section. Moreover, the tubular passage with a small diameter appropriately adjusts the rate of change in the liquid level (rate of rising of the liquid level). Consequently, it becomes possible that workers easily confirm with their eyes completion of collecting initial flow blood, collection of a required minimum amount of initial flow blood, and a variation in the amount of collected blood at the level of several ml from a change in the liquid level.

Further, in the blood sample container, the exhaust section preferably has an indicating section that indicates a predetermined liquid level of the initial flow blood.

With the above configuration, the provision of the indicating section that indicates a predetermined liquid level of initial flow blood enables workers to confirm more easily with their eyes collection of a required minimum amount of initial flow blood and a variation in the amount of collected blood at the level of several ml from a change in the liquid level.

Furthermore, in the blood sample container, it is preferable the exhaust section further includes an antifoaming filter at a position of the bacteria-impermeable filter near the internal space.

With the above configuration, since the provision of the antifoaming filter eliminates air bubbles in the initial flow blood if contained in the blood, the bacteria-impermeable filter is not air-blocked by blood membranes caused by foaming of the initial flow blood. Consequently, flowing of the initial flow blood does not automatically stop by the air-blocking before a required minimum amount of the initial flow blood is secured.

Moreover, the blood sample container further includes a blood collecting section communicating with the blood outflow port, that collects the initial flow blood, the blood collecting section including: a needle assembly having a hollow needle communicating with the blood outflow port; and a cylindrical holder having one end and the other end, the one end having the needle assembly mounted thereto and the other end having an opening in which a testing instrument that stores the initial flow blood is to be inserted.

With the above configuration, the provision of the blood collecting section enables the initial flow blood stored in the container body section to easily flow through the needle assembly into the testing instrument (a decompression blood collecting tube, for example) inserted in the holder.

A blood collecting instrument according to the present invention includes: a blood collecting needle; a storage section storing blood collected through the blood collecting needle; a blood collecting line connecting the blood collecting needle and the storage section together; a branch line branching from the middle of the blood collecting line; and the blood sample container communicating with the blood collecting line via the branch line.

With the above configuration, the provision of the blood sample container enables workers to easily confirm with their eyes collection of a required minimum amount of initial flow blood, as well as a variation in the amount of collected blood at the level of several ml from a change in the liquid level.

The storage section of the blood collecting instrument is preferably a blood bag or a blood separator.

With the above configuration, the storage section that is a blood bag or a blood separator can be used as a blood bag system or an apheresis collection set.

### Advantageous Effects of Invention

The blood sample container and the blood collecting instrument according to the present invention enable workers to easily recognize the completion of collecting initial flow blood, secure a required minimum amount of initial flow blood, and fine-adjust the amount of initial flow blood to be collected at the level of several ml according to individual donors.

### Brief Description of Drawings

FIG. 1 is a cross-sectional view illustrating the configuration of a blood sample container according to an embodiment of the present invention.
FIG. 2 (a) to FIG. 2(c) are perspective views illustrating the configuration of an edge part of an exhaust section.
FIG. 3 is a cross-sectional view illustrating the configuration of a blood sample container according to another embodiment.
FIG. 4 is a schematic view illustrating the configuration of a blood collecting instrument according to the embodiment.

### Description of Embodiments

A blood sample container according to an embodiment of the present invention will be described in detail with reference to the drawings.

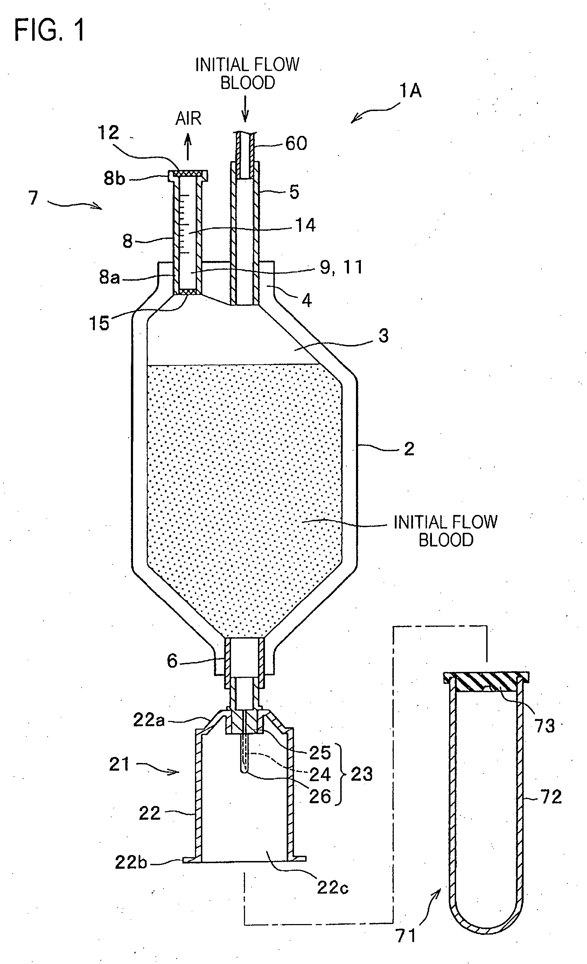

As shown in FIG. 1, a blood sample container 1A has a container body section 2, a blood inflow port 5, a blood outflow port 6, and an exhaust section 7.

The container body section 2 has an internal space 3 that stores (collects) initial flow blood to be used for testing in the internal space 3. Further, the container body section 2 is desirably in the shape of a bag, and is formed of layered sheet material of flexible resin such as polyvinyl chloride and a sealing section 4 arranged on the sheet material at the outer edges thereof by fusing (thermal fusing, high-frequency fusing) or adhesion. The internal space 3 is formed to be large enough for storing blood in a predetermined amount (for example, 25 ml).

In the container body section 2, preferably in the edges of the container body section 2, a blood inflow port 5, a blood outflow port 6, and an exhaust section 7 are provided so as to communicate with the internal space 3. FIG. 1 illustrates the blood sample container 1A when initial flow blood is to be drawn into the blood sample container 1A. At the upper edge of the container body section 2, the blood inflow port 5 and the exhaust section 7 are arranged, while at the lower edge, the blood outflow port 6 is arranged. However, when the blood sample container 1A is used in a manner that it is hung from a stand, for example, in a downward direction, the blood inflow port 5 and the blood outflow port 6 may be arranged at positions other than the position shown in FIG. 1 as long as the exhaust section 7 is arranged at the upper edge of the container body section 2. Specifically, although not shown in the drawings, the blood sample container 1A may be so configured that the blood inflow port 5 is arranged at the lower edge or the blood outflow port 6 is arranged at the upper edge of the blood sample container 1A.

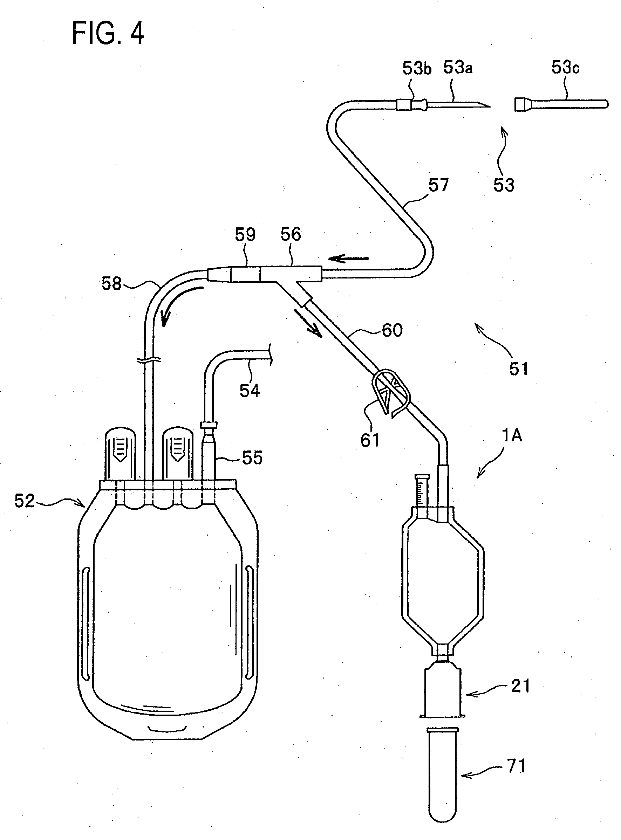

The blood inflow port 5 allows initial flow blood to flow into the container body section 2 by communicating with the internal space 3 of the container body section 2, and is made of a tubular member (tube) of flexible resin such as polyvinyl chloride. The blood inflow port 5 is supported at its one edge by the sheet material when the container body section 2 is prepared, and is bonded at the edge to the sealing section 4 of the container body section 2 by fusing or adhesion so as to communicate with the internal space 3. On the other hand, the blood inflow port 5 is bonded at its other edge to a branch tube 60 of a blood collecting instrument 51 by fusing or adhesion (see FIG. 4).

The blood outflow port 6 allows initial flow blood stored in the internal space 3 to flow out into a testing instrument, for example, by communicating with the internal space 3 of the container body section 2, and is made of a tubular member (tube) of flexible resin such as polyvinyl chloride. The blood outflow port 6 is supported at its one edge by the sheet material when the container body section 2 is prepared, and is bonded at the edge to the sealing section 4 of the container body section 2 by fusing or adhesion so as to communicate with the internal space 3. On the other hand, the blood outflow port 6 is preferably bonded at its other edge to a blood collecting section 21 described later by fusing or adhesion. It is to be noted that although not shown in the drawing, a needle assembly 23 (blood collecting section 21) described later has a hub 25 and one edge of the hub 25 may be used as the blood outflow port 6 instead of the tubular member (tube).

The exhaust section 7, which communicates with the internal space 3 of the container body section 2 and has a hydrophilic and bacteria-impermeable filter 12, allows air in the internal space 3 to be discharged. Further, the exhaust section 7 has a small-diameter tubular passage 11 for entry of a predetermined amount of initial flow blood. The tubular passage 11 is made of a tubular member (tube) 8 of rigid resin such as polycarbonate, which makes it easier to confirm initial flow blood that gets in contact with the bacteria-impermeable filter 12 after the liquid level of the initial flow blood stably rises. The tube 8 is preferably made of a transparent material, which enables easy confirmation of the liquid level of the initial flow blood.

The tube 8 is supported at its one edge 8a by the sheet material when the container body section 2 is prepared, and is bonded at the edge 8a to the sealing section 4 of the container body section 2 by fusing or adhesion so as to communicate with the internal space 3, and has an opening to the outside (atmosphere) at its other edge 8b. Note that although not shown in the drawings, the tube 8 may be made of a connection tube having a flexible tube arranged at one end of the connection tube and a rigid tube arranged at the other end, the flexible and rigid tubes being connected together, the flexible tube being made of polyvinyl chloride, for example, having properties similar to those of the container body section 2, which is easily fused or adhered, and the rigid tube being made of polycarbonate, for example, mentioned above. Furthermore, the hydrophilic and bacteria-impermeable filter 12 is arranged at the other edge 8b of the tube 8. The tube 8 is arranged at the upper edge of the container body section 2 when it is used in a manner such that the blood outflow port 6 faces in a vertical direction.

Since the exhaust section 7 includes a first tubular passage 9 (tubular passage 11) made of the tube 8, part of initial flow blood enters the first tubular passage 9 from the internal space 3 at the completion of the collection of the initial flow blood. With this inflow of the initial flow blood into the first tubular passage 9, the air having moved to the upper area of the internal space 3 in association with the inflow of the initial flow blood is discharged from the first tubular passage 9 to the outside (atmosphere) through the hydrophilic and bacteria-impermeable filter 12. At the same time, inflow of the initial flow blood to the exhaust section 7 causes a change in the liquid level in the first tubular passage 9. Because the first tubular passage 9 has a small diameter, the liquid level changes (rises) at an appropriate rate, and therefore it becomes easier for workers to confirm with their eyes completion of collecting the initial flow blood, securing of a required minimum amount of initial flow blood, variations in the amount of collected blood among donors by the level of several ml. It is when initial flow blood becomes in contact with the hydrophilic and bacteria-impermeable filter 12 that the collection of the initial flow blood is completed.

The first tubular passage 9 is preferably 1 ml to 10 ml in capacity and 1 mm to 20 mm in inner diameter and is more preferably 5 ml to 10 ml in capacity and 6 mm to 20 mm in inner diameter in order to response to variations in collected blood at the level of several ml among donors so that the exhaust section 7 can efficiently perform the operations. If the first tubular passage 9 is less than 1 ml in capacity and larger than 20 mm in inner diameter, it is difficult to deal with variations in collected blood among donors, in other words, to confirm variations in collected blood from changes in the liquid level. On the other hand, if the first tubular passage 9 is larger than 10 ml in capacity and less than 1 mm in inner diameter, the blood sample container 1A is difficult to use in many cases due to the first tubular passage 9 with an unnecessarily large length.

The inner diameter of the first tubular passage 9 will be further described. Each donor has his/her own unique blood flow rate in a range of approximately 0.3 ml to 1 ml per second. If the first tubular passage 9 has an inner diameter of 20 mm, the liquid level in the first tubular passage 9 rises by approximately 1 mm per second for a donor with a blood flow rate of 0.3 ml per second and rises by approximately 3 mm per second for a donor with a blood flow rate of 1 ml per second. Moreover, if the first tubular passage 9 has an inner diameter of 10 mm, the liquid level in the first tubular passage 9 rises by approximately 4 mm per second for a donor with a blood flow rate of 0.3 ml per second and rises by approximately 13 mm per second for a donor with a blood flow rate of 1 ml per second. Since it is difficult to visually confirm a change in the liquid level if the liquid level rises by less than 1 mm per second, the first tubular passage 9 preferably has an inner diameter of not larger than 20 mm.

The hydrophilic and bacteria-impermeable filter 12 is preferably made of hydrophilic porous material or hydrophilic nonwoven fabric, for example, with a strength capable of enduring a venous pressure so that the exhaust section 7 can carry out the operations efficiently. Since the bacteria-impermeable filter 12 is hydrophilic, it has gas-permeable (air-permeable) and liquid-impermeable (initial flow blood-impermeable) properties. Hence, initial flow blood automatically stops entering when it becomes in contact with the bacteria-impermeable filter 12. Moreover, since the bacteria-impermeable filter 12 is hydrophilic, it has a property of blocking air inflow when the initial flow blood is in contact with the bacteria-impermeable filter 12. The property contributes to interrupting airflow from the bacteria-impermeable filter 12 and maintaining the air tightness of the internal space 3. Therefore, when initial flow blood is collected by connecting a decompression blood collecting tube 71 (testing instrument) to a blood collecting section 21 described later, the decompression blood collecting tube 71 can have a maintained decompression state and drawing is ensured of initial flow blood into the decompression blood collecting tube 71. Examples of the bacteria-impermeable filter 12 include not only filters made of porous material and nonwoven fabric of hydrophilic material such as acrylate resin, but also filters made of porous material and nonwoven fabric coated at their surfaces with hydrophilic material such as acrylate resin by plasma processing, for example. The pore sizes of the bacteria-impermeable filter 12 are desirably in a range of 0.01 µm to 100 µm. The airflow is insufficient when the pore sizes are smaller than 0.01 µm and bacteria permeation is difficult to prevent when the pore sizes exceed 100 µm.

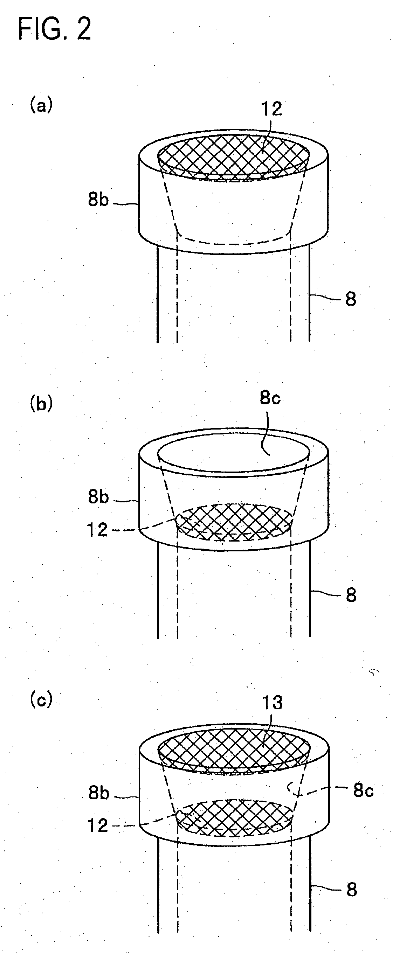

Although the hydrophilic and bacteria-impermeable filter 12 is arranged at the tip of the other edge 8b of the tube 8 having an opening to the outside (atmosphere) as shown in FIG. 2(a), it is desirable that a concave portion 8c is provided in the tip of the other edge 8b and the bacteria-impermeable filter 12 is arranged on the bottom of the concave portion 8c, as shown in FIG. 2(b). Further, as shown in FIG. 2(c), a configuration may be employed in which the bacteria-impermeable filter 12 is provided on the bottom of the concave portion 8c provided in the tip of the other edge 8b and the tip of the other edge 8b (the upper surface of the concave portion) is covered with a covering member 13 made of nonwoven fabric, for example. Workers can be prevented from touching the bacteria-impermeable filter 12 by mistake by forming the bacteria-impermeable filter 12 of the exhaust section 7 (tube 8) as shown in FIGS. 2 (b) and 2(c), and this makes it possible to avoid deterioration of an air discharge function of the bacteria-impermeable filter 12, which is caused when the bacteria-impermeable filter 12 is touched by the workers and gotten wet.

Next, another embodiment of the blood sample container will be described with reference to the drawings.

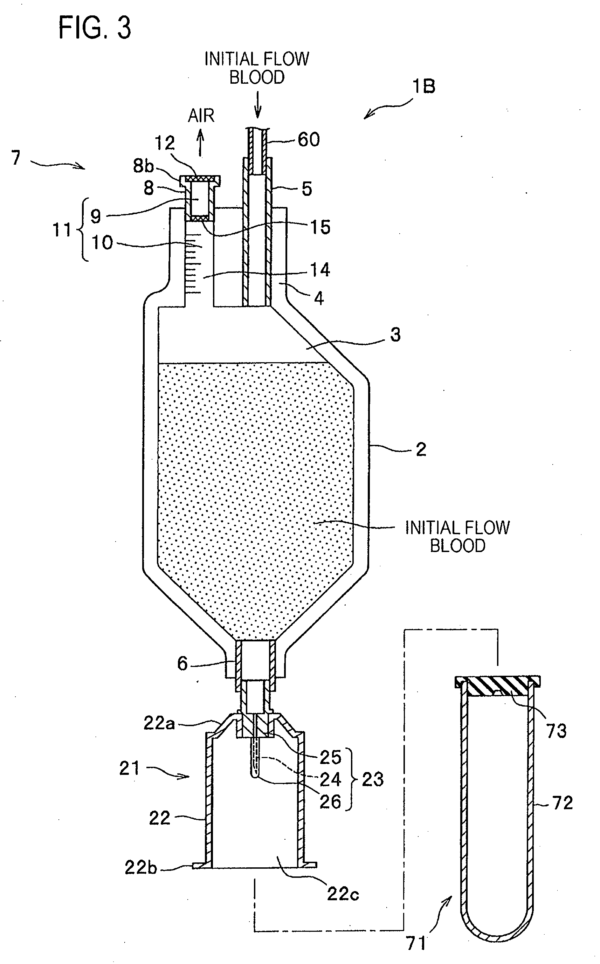

As shown in FIG. 3, a blood sample container 1B is configured so that the tubular passage 11 of the exhaust section 7 includes a first small-diameter tubular passage 9 made of the tube 8 and a second small-diameter tubular passage 10 made of an upward protruding portion of the top region of the internal space 3 so as to communicate with the first small-diameter tubular passage 9. The first tubular passage 9 is similar in configuration to the tubular passage 11, which is made of the tube 8 having one edge 8a and the other edge 8b, the edge 8a being bonded to the sealing section 4 of the container body section 2 by fusing or adhesion so as to communicate with the internal space 3 and the other edge 8b having an opening to the outside (atmosphere), as shown in FIG. 1. The hydrophilic and bacteria-impermeable filter 12 is arranged at the other edge 8b of the tube 8.

The second tubular passage 10 is formed by adjusting the shape and the size of the sealing section 4 when the container body section 2 is prepared by fusing or adhesion of the sheet. The operations of the second tubular passage 10 are similar to those of the first tubular passage 9. Further, the tubular passage 11 (the first tubular passage 9 and the second tubular passage 10) is desirably 1 ml to 10 ml in capacity and 1 mm to 20 mm in inner diameter. Although not shown in the drawings, the second tubular passage 10 may be formed into a tapered shape with its inner diameter becoming smaller in a direction of the first tubular passage 9 of the tube 8.

Further, although not shown in the drawings, the blood sample container may be configured so that the tubular passage 11 of the exhaust section 7 is formed of the second small-diameter tubular passage 10 only, which is made of an upward protruding portion of the top region of the internal space 3. The hydrophilic and bacteria-impermeable filter 12 is arranged at the upper edge of the second tubular passage 10.

As shown in FIGS. 1 and 3, in the blood sample containers 1A and 1B, it is desirable that the exhaust section 7 has an indicating section 14 that indicates a predetermined liquid level of initial flow blood.

The indicating section 14 has marks or a scale, for example, that indicates the liquid level showing that initial flow blood drawn into the container body section 2 has reached a required minimum amount, or the amount of change in the liquid level (for example, 0.3 ml to 5 ml). Further, it is desirable that the indicating section 14 is included in the first tubular passage 9 made of the tube 8 in the blood sample container 1A, as well as in the second tubular passage 10 made of a portion of the internal space 3 in the blood sample container 1B.

As shown in FIGS. 1 and 3, in the blood sample containers 1A and 1B, the exhaust section 7 preferably has an antifoaming filter 15 at a position of the bacteria-impermeable filter 12 near the internal space 3. Further, the antifoaming filter 15 is desirably arranged at a position near the boundary between the first tubular passage 9 (second tubular passage 10) and the internal space 3.

Examples of preferred materials of the antifoaming filter 15 include various types of porous materials such as expanded materials (for example, expanded polyurethane, expanded polyethylene, expanded polypropylene, and expanded polystyrene), mesh, woven fabric, nonwoven fabric, and sintered materials (porous ceramics and resins). The pore sizes of the antifoaming filter 15 are preferably in a range of approximately 20 µm to 10 mm, in particular, approximately 50 µm to 5 mm. Moreover, the antifoaming filter 15 desirably contains, in the porous material, for example, an antifoaming agent provided with a function of breaking foams that get in contact with the antifoaming filter 15. As the antifoaming agent, silicon (a compound type with silica mixed, an oil type, for example) is preferable. The porous material is allowed to contain the antifoaming agent in a manner that the porous material is immersed into a liquid containing the antifoaming agent or in a manner that a liquid containing the antifoaming agent is applied or sprayed to the porous material and is dried.

As is shown in FIGS. 1 and 3, it is preferable that the blood sample containers 1A and 1B further include the blood collecting section 21 communicating with the blood outflow port 6, which allows initial flow blood stored in the container body section 2 to be collected into the decompression blood collecting tube 71 (testing instrument).

The blood collecting section 21 includes a needle assembly 23 and a holder 22. The needle assembly 23 has a hollow needle 24 made of a metal with a sharp needle tip at its end and a rigid resin, for example, a hub 25 made of polyolefin, for example, fixed to the base end part of the hollow needle 24, and a rubber casing 26 covering the hollow needle 24. Moreover, the hub 25 is bonded to the blood outflow port 6, whereby the hollow needle 24 communicates with the blood outflow port 6. The holder 22 is a cylindrical member made of, for example, polyolefin and has one end 22a and the other end 22b, the one end 22a having the needle assembly 23 placed therein and the other end 22b having an opening 22c in which the decompression blood collecting tube 71 (testing instrument) is to be inserted. The one end 22a of the holder 22 is set near the outer periphery of the hollow needle 24 of the needle assembly 23 and is bonded to the hub 25 concentrically with the hollow needle 24.

Next, a blood collecting instrument according to an embodiment of the present invention will be described.

As shown in FIG. 4, a blood collecting instrument 51 (blood bag system) includes: a blood collecting needle 53; a blood collecting bag 52 (storage section or blood bag) storing blood collected through the blood collecting needle 53; a blood collecting line connecting the blood collecting needle 53 and the blood collecting bag 52 together; a branch line branching from the middle of the blood collecting line; and the blood sample container 1A communicating with the blood collecting line via the branch line for collecting (storing) initial flow blood. Each of the components will be hereinafter described. The description of the blood sample container 1A will be omitted since it has been previously described. The blood collecting instrument 51 may include the blood sample container 1B instead of the blood sample container 1A.

The blood collecting needle 53 has a hollow needle 53a made of a metal with a sharp needle tip at its end and a rigid resin, for example, a hub 53b made of polyolefin, for example, fixed to the base end part of the hollow needle 53a, and a protector 53c of a rigid resin, for example, covering the hollow needle 53a.

The blood collecting bag 52 is a bag-shaped container obtained by stacking resin sheets of polyvinyl chloride, for example, on one another and fusing or adhering the sheets at the outer edges thereof, and contains blood that is left after initial flow blood is removed (collected) from blood collected by the blood collecting needle 53 using a blood processing method described later. Further, the blood collecting bag 52 preferably contains an anticoagulant in advance.

A tube 54 is connected at its one end to the upper part of the blood collecting bag 52 via a sealing member 55, and is connected at its other end to a leukocyte removal filter and blood bags such as a withdrawing bag (red blood cell collecting bag), a blood plasma collecting bag, and a bag containing a red blood cell storage solution, not shown in the drawings. A publicly-known filter is used as the leukocyte removal filter, and bag-shaped containers made of polyvinyl chloride, for example, similar to the blood collecting bag 52 are used as the withdrawing bag, the blood plasma collecting bag, and the bag containing a red blood cell storage solution.

A tube 58 is connected at its one end to the upper part of the blood collecting bag 52 and is connected at its other end to a branch connector 56, which is connected to the blood collecting needle 53 (hub 53b) via a tube 57. Hence, the tubes 57 and 58 and the branch connector 56 form the blood collecting line, and the blood collecting bag 52 and the blood collecting needle 53 are connected together by the blood collecting line. Moreover, the branch tube 60 is connected to the blood sample container 1A at its one end and to the branch connector 56 at its other end, the branch connector 56 forms the branch line branching from the middle of the blood collecting line, and the blood sample container 1A communicates with the blood collecting line via the branch line. Further, a sealing member 59 is provided between the branch connector 56 and the tube 58 so that initial flow blood can be prevented from flowing into the tube 58 near the blood collecting bag 52 when the initial flow blood is collected (stored) into the blood sample container 1A by the branch tube 60. Moreover, the branch tube 60 includes a clamp 61 to close the blood collecting tube for collecting initial flow blood.

The sealing members 55 and 59 are each made of a short tube and a cylindrical body having a solid end part contained therein so as to close the tube, which are not shown in the drawings. The passage in the short tube is opened by breaking the solid end part of the cylindrical body. The short tube is made of flexible material such as polyvinyl chloride, and the cylindrical body is made of rigid material such as polycarbonate. The branch connector 56, the tubes 54, 57, 58, and the branch tube 60 are made of, for example, polyvinyl chloride.

Next, a blood processing method using the blood sample container and the blood collecting instrument will be described. The configuration of the blood sample container will be described with reference to FIGS. 1 and 3 as appropriate.

First, as shown in FIG. 4, in the blood collecting instrument 51, since the clamp 61 is in a closed state, the blood collecting tube of the branch tube 60 is in an open state. The blood collecting tube of the tube 58 is in a closed state by the sealing member 59. Further, the blood sample container 1A is hung from a stand, for example, in a downward direction and the exhaust section 7 is located in the upper part of the blood sample container 1A. This applies equally to the blood sample container 1B.

When the blood collecting needle 53 is tapped into a vein of a donor in the above state, initial flow blood is drawn into the branch tube 60 through the blood collecting needle 53, the tube 57, and the branch connector 56 and is collected (stored) into the blood sample container 1A. In this case, since the blood collecting tube of the tube 58 is blocked by the sealing member 59 as described above, the initial flow blood is surely introduced into the branch tube 60 from the tube 57 through the branch connector 56. Further the air in the tube 57, the branch connector 56, and branch tube 60 is discharged into the blood sample container 1A (container body section 2) prior to entry of the initial flow blood. This applies equally to the blood sample container 1B.

In the blood sample container 1A, initial flow blood is drawn from the blood inflow port 5 into the internal space 3 of the container body section 2, as shown in FIG. 1. With the inflow of the initial flow blood into the internal space 3, the air in the internal space 3 is collected in the upper region thereof and is discharged to the outside (atmosphere) through the hydrophilic and bacteria-impermeable filter 12 of the exhaust section 7. Further, part of initial flow blood that has flown into the internal space 3 is drawn into the first small-diameter tubular passage 9 of the exhaust section 7 at the completion of the collecting the initial flow blood. The drawn initial flow blood rises at an appropriate rate in the first tubular passage 9. Further, a worker confirms with his/her eyes that the liquid level in the first tubular passage 9 has reached the liquid level of a required minimum amount of initial flow blood to be collected or the liquid level of initial flow blood to be collected in an amount determined in advance according to a donor, or confirms with his/her eyes that the liquid level in the first tubular passage 9 has become in contact with the hydrophilic and bacteria-impermeable filter 12. Thereafter, the worker closes the clamp 61 to close the blood collecting tube of the branch tube 60 and stops entry of the initial flow blood from the blood inflow port 5. In the blood sample container 1B, part of initial flow blood flows into the small-diameter tubular passage 11 (first tubular passage 9 and second tubular passage 10) and the liquid level in the tubular passage 11 is confirmed, as shown in FIG. 3.

Subsequently, blood collection into the blood collecting bag 52 (claimed blood collection) is started.

A tube in the sealing member 59 is opened by breaking the solid end part of the sealing member 59 (not shown in the drawings) in this case. This operation opens the blood collecting tube of the tube 58, whereby the tubes 57 and 58 communicate with each other. Thus, the collected blood flows into the blood collecting bag 52 through the tube 57, the branch connector 56, the sealing member 59, and the tube 58.

Moreover, a sample of the initial flow blood collected (stored) into the blood sample container 1A is obtained for a testing instrument such as the decompression blood collecting tube 71 in conjunction with blood collection into the blood collecting bag 52, if the sampling is judged as possible from the condition of a donor or blood collection.

In order for the sampling to be performed, the decompression blood collecting tube 71 is inserted into the blood collecting section 21 (holder 22) until it reaches the deepest point of the holder 22 and the hollow needle 24 is injected in a rubber sheath 73 set in a blood collecting tube body 72 of the decompression blood collecting tube 71, as shown in FIG. 1. In this way, the initial flow blood stored in the blood sample container 1A is drawn into the blood collecting tube body 72 and is sampled. Thereafter, the decompression blood collecting tube 71 is removed from the blood collecting section 21 (holder 22). It is to be noted that the operation is repeated if the sampling of the initial flow blood is to be performed on more than one decompression blood collecting tube 71. This applies equally to the blood sample container 1B.

Subsequently, as shown in FIG. 4, in the blood collection into the blood collecting bag 52, after blood is collected into the blood collecting bag 52 in an amount set in advance, the blood collecting needle 53 is drawn from a vein of a donor, and the tube 58 and the branch tube 60 are closed and sealed if needed by a tube sealer, for example. Thereafter, the blood sample container 1A and the blood collecting needle 53 are separated. In this way, the blood collecting bag 52 can be obtained that contains blood that is left after the initial flow blood has been removed. This applies equally to the blood sample container 1B.

In the meanwhile, the blood contained in the blood collecting bag 52 is filtered by the leukocyte removal filter not shown in the drawings so that white blood cells and platelets are separated from the blood, the other blood components are collected into the withdrawing bag, and the blood collecting bag 52 and the leukocyte removal filter are separated from each other. Moreover, the blood in the withdrawing bag is centrifuged so as to be divided into red blood cell layers and blood plasma layers, blood plasma is transferred into the blood plasma bag, and a red blood cell storage solution in the bag containing the red blood cell storage solution is added to and mixed with concentrated red cells that are left in the withdrawing bag.

Although the embodiments of the blood sample container and the blood collecting instrument have been described, the present invention is not limited to the embodiments. Specifically, the blood collecting instrument according to the present invention may use as the storage section a blood separator (such as a centrifugal separator and a membrane separator) instead of a blood bag. In other word, the blood collecting instrument is not limited to a blood bag system and may be an apheresis collection set.

### Reference Signs List

- 1A, 1B: blood sample container
- 2: container body section
- 3: internal space
- 5: blood inflow port
- 6: blood outflow port
- 7: exhaust section
- 9: first tubular passage
- 10: second tubular passage
- 11: tubular passage
- 12: bacteria-impermeable filter
- 51: blood collecting instrument
- 52: blood collecting bag (storage section)
- 53: blood collecting needle

## Claims

1. A blood sample container (1A; 1B) comprising:
a container body section (2) having an internal space (3) for storing initial flow blood to be used for testing;
a blood inflow port (5) communicating with the internal space (3), that allows the initial flow blood to flow in;
a blood outflow port (6) communicating with the internal space (3), that allows the stored initial flow blood to flow out; and
an exhaust section (7) communicating with the internal space (3) through a first end and being capable of discharging air at a second end and having a hydrophilic and bacteria-impermeable filter (12), that allows air in the internal space (3) to be discharged, wherein
the exhaust section (7) is positioned at an upper end of the container body section (2) when the blood sample container (1A; 1B) is used in a manner such that the blood outflow port (6) faces in a vertical direction, and has a small-diameter tubular passage (9) that allows the air in the internal space (3) to be discharged by a predetermined amount of the initial flow blood that has flown into the tubular passage (9)
**characterized in that**
the exhaust section (7) has an anti-foaming filter (15) arranged at the first end and has the hydrophilic and bacteria-impermeable filter (12) arranged at the second end, and **in that**
the small-diameter tubular passage (9) is arranged between the first and second ends of the exhaust section (7).

2. The blood sample container (1B) according to claim 1, wherein
the tubular passage (9) is at least one of a first small-diameter tubular passage (9) and a second small-diameter tubular passage (10), the first tubular passage (9) being formed of a tube connected to the container body section (2) and the second tubular passage (10) being formed of a portion of the internal space (3) which projects in an upward direction.

3. The blood sample container (1A; 1B) according to claim 1, wherein the exhaust section (7) has an indicating section (14) that indicates a predetermined liquid level of the initial flow blood.

4. The blood sample container (1A; 1B) according to claim 1, wherein the exhaust section (7) further includes an anti-foaming filter (15) at a position of the bacteria-impermeable filter (12) near the internal space (3).

5. The blood sample container (1A; 1B) according to claim 1, further comprising a blood collecting section (21) communicating with the blood outflow port (6), that collects the initial flow blood, wherein
the blood collecting section includes: a needle assembly (23) having a hollow needle (24) communicating with the blood outflow port (6); and a cylindrical holder (22) having one end and the other end, the one end having the needle assembly (23) mounted therein and the other end having an opening in which a testing instrument (71) that stores the initial flow blood is to be inserted.

6. A blood collecting instrument (51), comprising:
a blood collecting needle (53);
a storage section (52) storing blood collected through the blood collecting needle;
a blood collecting line (57, 56, 59, 58) connecting the blood collecting needle (53) and the storage section (52) together;
a branch line (60) branching from the middle of the blood collecting line (57, 56, 59, 58); and
the blood sample container (1A; 1B) according to one of claims 1 to 5 communicating with the blood collecting line (57, 56, 59, 58) via the branch line (60).

7. The blood collecting instrument (51) according to claim 6, wherein the blood storage section is a blood bag (52) or a blood separator.

## Patentansprüche

1. Blutprobenbehälter (1A; 1B) mit:
einem Behälterkörperabschnitt (2), der einen Innenraum (3) zum Lagern eines Anfangsblutstroms aufweist, der zum Untersuchen zu verwenden ist;
einem Bluteinströmanschluss (5), der mit dem Innenraum (3) in Verbindung ist, der dem Anfangsblutstrom das Einströmen ermöglicht;
einem Blutausströmanschluss (6), der mit dem Innenraum (3) in Verbindung ist, der dem gelagerten Anfangsblutstrom das Ausströmen ermöglicht; und
einem Auslassabschnitt (7), der mit dem Innenraum (3) durch ein erstes Ende in Verbindung ist und in der Lage ist, Luft an einem zweiten Ende abzugeben, und der einen hydrophilen und bakterienundurchlässigen Filter (12) aufweist, der ein Abgeben der Luft in dem Innenraum (3) ermöglicht, wobei
der Auslassabschnitt (7) an einem oberen Ende des Behälterkörperabschnitts (2) positioniert ist, wenn der Blutprobenbehälter (1A; 1B) in einer Weise derart verwendet wird, dass der Blutausströmanschluss (6) in eine vertikale Richtung gerichtet ist, und einen rohrförmigen Durchtritt (9) kleinen Durchmessers aufweist, der es der Luft in dem Innenraum (3) durch eine vorbestimmte Menge des Anfangsblutstroms, der in den rohrförmigen Durchtritt (9) geströmt ist, ermöglicht, abgegeben zu werden,
**dadurch gekennzeichnet, dass**
der Auslassabschnitt (7) einen Schaumverhinderungsfilter (15) aufweist, der an dem ersten Ende angeordnet ist, und den hydrophilen und bakterienundurchlässigen Filter (12) an dem zweiten Ende angeordnet aufweist, und dadurch, dass
der rohrförmige Durchtritt (9) kleinen Durchmessers zwischen den ersten und zweiten Enden des Auslassabschnitts (7) angeordnet ist.

2. Blutprobenbehälter (1B) nach Anspruch 1, wobei
der rohrförmige Durchtritt (9) zumindest einer eines ersten rohrförmigen Durchtritts (9) kleinen Durchmessers und eines zweiten rohrförmigen Durchtritts (10) kleinen Durchmessers ist, wobei der erste rohrförmige Durchtritt (9) aus einem Rohr ausgebildet ist, das mit dem Behälterkörperabschnitt (2) verbunden ist, und der zweite rohrförmige Durchtritt (10) aus einem Abschnitt des Innenraums (3) ausgebildet ist, der in einer Richtung nach oben vorspringt.

3. Blutprobenbehälter (1A; 1B) nach Anspruch 1, wobei der Auslassabschnitt (7) einen Anzeigeabschnitt (14) aufweist, der eine vorbestimmte Flüssigkeitshöhe des Anfangsblutstroms anzeigt.

4. Blutprobenbehälter (1A; 1B) nach Anspruch 1, wobei der Auslassabschnitt (7) außerdem einen Schaumverhinderungsfilter (15) an einer Position des bakterienundurchlässigen Filters (12) nahe des Innenraums (3) hat.

5. Blutprobenbehälter (1A; 1B) nach Anspruch 1, außerdem mit einem Blutsammelabschnitt (21), der mit dem Blutausströmanschluss (6) in Verbindung ist, der den Anfangsblutstrom sammelt, wobei
der Blutsammelabschnitt hat: eine Nadelbaugruppe (23), die eine hohle Nadel (24) aufweist, die mit dem Blutausströmanschluss (6) in Verbindung ist; und einen zylindrischen Halter (22), der ein Ende und das andere Ende aufweist, wobei das eine Ende die Nadelbaugruppe (23) darin montiert aufweist, und das andere Ende eine Öffnung aufweist, in der ein Prüfinstrument (71), welches den Anfangsblutstrom lagert, einzufügen ist.

6. Blutsammelinstrument (51), mit:
einer Blutsammelnadel (53);
einem Lagerabschnitt (52), der durch die Blutsammelnadel gesammeltes Blut lagert;
einer Blutsammelleitung (57, 56, 59, 58), die die Blutsammelnadel (53) und den Speicherabschnitt (52) miteinander verbindet;
eine Zweigleitung (60), die von der Mitte der Blutsammelleitung (57, 56, 59, 58) abzweigt; und
dem Blutprobenbehälter (1A; 1B) nach einem der Ansprüche 1 bis 5, der mit der Blutsammelleitung (57, 56, 59, 58) über die Zweigleitung (60) in Verbindung ist.

7. Blutsammelinstrument (51) nach Anspruch 6, wobei der Blutspeicherabschnitt ein Blutbeutel (52) oder ein Blutseparator ist.

## Revendications

1. Récipient d'échantillon de sang (1A ; 1B) comprenant :
une section de corps de récipient (2) ayant un espace interne (3) pour stocker l'écoulement de sang initial destiné être utilisé pour analyse ;
un orifice d'entrée de sang (5) communiquant avec l'espace interne (3) et qui permet à l'écoulement initial de sang de s'écouler dans ce dernier ;
un orifice de sortie de sang (6) communiquant avec l'espace interne (3) et qui permet à l'écoulement initial de sang stocké de sortir par ce dernier ; et
une section d'échappement (7) communiquant avec l'espace interne (3) par le biais d'une première extrémité et pouvant décharger l'air au niveau d'une seconde extrémité et ayant un filtre hydrophile et imperméable aux bactéries (12), qui permet de décharger l'air dans l'espace interne (3), dans lequel
la section d'échappement (7) est positionnée au niveau d'une extrémité supérieure de la section de corps de récipient (2) lorsque le récipient d'échantillon de sang (1A ; 1B) est utilisé de sorte que l'orifice de sortie de sang (6) est orienté dans une direction verticale, et a un passage tubulaire de petit diamètre (9) qui permet de décharger l'air dans l'espace interne (3) selon une quantité prédéterminée de l'écoulement initial de sang qui s'est écoulé dans le passage tubulaire (9),
**caractérisé en ce que**
la section d'échappement (7) a un filtre antimousse (15) agencé au niveau de la première extrémité et a le filtre hydrophile et imperméable aux bactéries (12) agencé au niveau de la seconde extrémité, et **en ce que**
le passage tubulaire de petit diamètre (9) est agencé entre les première et seconde extrémités de la section d'échappement (7).

2. Récipient d'échantillon de sang (1B) selon la revendication 1, dans lequel
le passage tubulaire (9) est au moins l'un parmi un premier passage tubulaire de petit diamètre (9) et un second passage tubulaire de petit diamètre (10), le premier passage tubulaire (9) étant formé avec un tube raccordé à la section de corps de récipient (2) et le second passage tubulaire (10) étant formé avec une partie de l'espace interne (3) qui fait saillie dans la direction ascendante.

3. Récipient d'échantillon de sang (1A ; 1B) selon la revendication 1, dans lequel la section d'échappement (7) a une section d'indication (14) qui indique un niveau de liquide prédéterminé de l'écoulement initial de sang.

4. Récipient d'échantillon de sang (1A ; 1B) selon la revendication 1, dans lequel la section d'échappement (7) comprend en outre un filtre antimousse (15) dans une position du filtre imperméable aux bactéries (12) à proximité de l'espace interne (3).

5. Récipient d'échantillon de sang (1A ; 1B) selon la revendication 1, comprenant en outre une section de collecte de sang (21) communiquant avec l'orifice de sortie de sang (6) qui collecte l'écoulement initial de sang, dans lequel
la section de collecte de sang comprend : un ensemble d'aiguille (23) ayant une aiguille creuse (24) communiquant avec l'orifice de sortie de sang (6) ; et un support cylindrique (22) ayant une extrémité et l'autre extrémité, la une extrémité ayant l'ensemble d'aiguille (23) monté à l'intérieur de cette dernière et l'autre extrémité ayant une ouverture dans laquelle un instrument d'analyse (71) qui stocke l'écoulement initial de sang doit être inséré.

6. Instrument de prélèvement de sang (51) comprenant :
une aiguille de prélèvement de sang (53) ;
une section de stockage (52) stockant le sang prélevé par l'aiguille de prélèvement de sang ;
une ligne de prélèvement de sang (57, 56, 59, 58) raccordant l'aiguille de prélèvement de sang (53) et la section de stockage (52) ensemble ;
une ligne de bifurcation (60) bifurquant à partir du centre de la ligne de prélèvement de sang (57, 56, 59, 58) ; et
le récipient de prélèvement de sang (1A ; 1B) selon l'une des revendications 1 à 5 communiquant avec la ligne de prélèvement de sang (57, 56, 59, 58) via la ligne de bifurcation (60).

7. Instrument de prélèvement de sang (51) selon la revendication 6, dans lequel la section de stockage de sang est une poche de sang (52) ou un séparateur de sang.
